# NEW EUROPEAN PATENT SPECIFICATION

(11) **EP 1 574 289 B2**
(45) Date of publication and mention of the opposition decision: **06.11.2019**
(45) Mention of the grant of the patent: 17.10.2012
(21) Application number: 05004803.2
(22) Date of filing: 04.03.2005
(51) Int. Cl.: B24B 37/04, B24B 41/06

(54) **A method for manufacturing a carrier for holding an object to be polished**
Verfahren zur Herstellung eines Trägers zum Halten eines zu polierenden Gegenstandes
Procédé pour la fabrication d' un support pour maintenir un objet à polir

(30) Priority: 09.03.2004 JP 2004066013
(43) Date of publication of application: 14.09.2005
(73) Proprietor: Speedfam Co., Ltd., Ayase, Kanagawa 252-1104 (JP)
(72) Inventor: Yoshida, Akira, Kanagawa-pref., 252-1123 (JP); Shimizu, Toshikuni, Kanagawa-pref., 252-1123 (JP)
(74) Representative: Beckmann, Claus

(56) References cited:
- WO-A1-01/34346
- DE-A1- 10 247 200
- DE-A1- 19 905 737
- US-A- 5 731 046
- US-A- 6 042 688

## Description

### BACKGROUND OF THE INVENTION

### Field of the Invention

The present invention relates to a method for manufacturing a carrier for holding an object to be polished (hereinafter referred to as "polishing object"), which is used in a double-sided polishing apparatus that simultaneously polishes both surface of the polishing object by holding the polishing object between upper and lower platens covered with abrasive cloth with the platens and the polishing object being in pressure contact with each other, and rotating at least one of the platens and the polishing object, the surface of the carrier being coated with diamond-like carbon.

### Description of the Related Art

In polishing a silicon wafer, a compound semiconductor wafer, an aluminum-made magnetic disk substrate, a glass-made magnetic disk substrate, or a polishing object that is made of photomask glass, a crystal oscillator, metal, or the like, the polishing object is held by a carrier cut into a shape where a holding hole is formed in conformity to a shape of the polishing object and peripheral (outer) gear teeth are arranged at an outer edge so as to mesh with an internal gear and sun gear of a double-sided polishing machine. The polishing object is driven together with the carrier. The carrier is mainly used as a member of a polishing apparatus for lapping or polishing both surfaces of a silicon wafer, a magnetic disk substrate, or the like at a time. Hence, the thickness of the carrier member is set slightly smaller than that of the polishing object such as a wafer.

Up to now, the following method has been generally used for producing such carriers. That is, glass cloth woven out of a metal material such as steel or stainless steel and glass fibers, or organic fiber cloth is impregnated with an epoxy resin, a phenol resin, or other such thermosetting resins. Then, several cloths are stacked into a desired thickness, pressed with a pressing machine, and cured with heat. The resultant material, for example, a so-called FRP is used as a raw material and cut into a size enough to hold the polishing object in accordance with the intended use. In addition, its peripheral portion is cut in conformity with a gear shape.

Those carriers are rotated together with a silicon wafer, a magnetic disk substrate, etc. in the polishing apparatus, and forcedly driven by an internal gear and a sun gear through its peripheral gear teeth. Hence, the surface is polished to some degree to lower its strength or accuracy of form, resulting in reduction in durability over time. Also, a gear portion is worn to cause the carrier to come off from the polishing apparatus or to destabilize polishing conditions. This requires replacement with new carriers each time or periodically. Accordingly, there is an increasing demand for development of carriers having high durability that secures as long a service life as possible. To meet such a demand, there is proposed an FRP-based carrier as disclosed in JP 2001-038609 A, for example. Furthermore, as a purity of a silicon wafer is increased, there has arisen a problem about contamination of the wafer with a trace amount of heavy metal eluting from a metal-made carrier, albeit being negligible in a conventional technique.

In the conventional technique, regarding the silicon wafer or compound wafer, the thickness is adjusted by lapping or grinding, and wafer is etched with an acid or alkali etchant, followed by polishing to attain a mirror-finished surface on one or both of front and rear sides of the wafer. With a view to increasing a surface accuracy or preventing house dust contamination, some objects to be polished, which include the silicon wafer, undergo the double-sided polishing, in which the polishing was hitherto performed on only side. To that end, a demand for double-sided polishing is growing. An indispensable condition for double-sided polishing is to use a carrier for holding a polishing object. In this case, what is important are an abrasion resistance and durability of a carrier itself, and whether or not heavy metal in the carrier causes any contamination.

A carrier made of, for example, a hard resin or a nonmetal material such as FRP is inferior in the abrasion resistance and durability, and thus falls short of practical applicability. Meanwhile, a carrier made of a metal material such as steel or stainless steel has a problem about the heavy metal contamination. Hence, there is a growing demand for development of carriers that will overcome all the problems.

As one possible method of preventing heavy metal contamination on the assumption that a metal material superior in abrasion resistance and durability is used as a substrate, the substrate surface is coated with any material. As an example thereof, there is proposed a ceramic-coated metal carrier as disclosed in JP 04-026177 A. Although exhibiting an extremely high performance regarding the abrasion resistance, the carrier has a problem in that ceramic particles adhering to the surface drop out of the surface, and the polishing object is scratched thereby. Also, JP 2002-018707 A discloses an SK steel-made carrier whose surface is plated with metal. This offers by no means a solution to the problem in that the polishing object is contaminated with heavy metal, in particular, in polishing the semiconductor wafer. Further, JP 11-010530 A discloses a carrier given a dressing function, which is prepared by welding the ceramic particles to uneven upper and lower surfaces and in addition, coating the uneven surfaces with diamond-like carbon. The document describes that the durability and the abration resistance are improved by the effect of the diamond-like carbon. The carrier is devised for the purpose of preventing the ceramic particles functioning to dress the target from coming off from the surface, and thus differs the carrier for holding the polishing object which is used for double-sided polishing.

The inventors of the present invention have made extensive studies with an aim to solve the problem regarding the durability, the abrasion resistance, and the heavy metal contamination in polishing both surfaces of the semiconductor material such as a silicon wafer. As a result, the inventors presupposed that the above-mentioned problems may be solved by coating an ordinary carrier with diamond-like carbon on its surface with high surface smoothness, and have worked on its development and research. As has been known in the art, a feature of the diamond-like carbon (hereinafter, referred to as "DLC") resides in its structure, albeit amorphous carbon structure, in which a diamond structure (SP3 bond) and a graphite structure (SP2 bond) coexist, and which has as high hardness as diamond, and attains high surface smoothness and low friction coefficient unlike diamond. The DLC can be easily formed into a thin film by plasma CVD etc. as disclosed in JP 10-046344 A. The inventors of the present invention have concluded that a carrier produced by uniformly and firmly lamination the DLC thin film of the above performance thereon and a manufacturing method therefore would solve the above-mentioned problems.

The inventors of the present invention have found, as a result of extensive studies on the problem inherent in the aforementioned conventional double-sided polishing carrier, all or part of the carrier surface is coated with the uniform DLC thin film having a highly smooth surface, whereby it is possible to remarkably improve the abrasion resistance and durability of the carrier, to prevent heavy metal from eluting from the carrier to cause contamination, and to protect the surface to be polished against any scratches resulting from a coating material coming off from the carrier surface.

Document US 6 042 688 A discloses a method for manufacturing a carrier for holding an object to be polished used for a double-sided polishing apparatus, the method comprising to provide a substrate with a certain thickness. For example, according to column 6, lines 8 to 11 of this reference where the finish thickness of the polished work is 725 µm, the thickness of the carrier exclusive of the projections should be 600 µm from the standpoint of the mechanical strength.

### SUMMRY OF THE INVENTION

It is therefore an object of the present invention to provide a manufacturing method for a carrier having high durability and abrasion resistance and causing as few scratches on an object to be polished (hereinafter referred to as "polishing object") and little heavy metal contamination as possible having the features of claim 1.

### DETALLED DESCRIPTION OF THE PREFERRED EMBODIMENT

Hereinafter, an embodiment of the present invention will be described. A carrier is shaped into a disk-like thin plate, and has a peripheral gear at its outermost edge and holding hole for holding an object to be polished (hereinafter referred to as "polishing object") in its inner position, and optionally a lightening hole. A carrier size varies depending on an apparatus model but ranges from several centimeters to several ten centimeters; some carriers exceed 1 m in size by the model. The carrier somewhat thinner than the polishing object is used. For example, silicon wafer has a thickness of about several hundred micrometers. Accordingly, the entire surface of a carrier substrate corresponds to the front and rear surfaces and a side surface (section along its thickness direction). An area to be coated with DLC includes the side surface. In particular, the carrier is thin in section along its thickness direction, and besides, meshes with an internal gear and sun gear of an apparatus or contacts the polishing object at the side surface portion. Hence, such a portion is most likely to receive a local stress and a large frictional force, and thus is more fragile than other portions. Even if not cracked, this portion will cause minute crack and heavy metal contamination resulting from wear. Upon polishing, a polishing object is held between upper and lower platens covered with abasive cloth and polished while spraying an abrasive such as colloidal silica slurry. As a result, although the front and rear surfaces of the carrier are not directly applied with pressure, they are polished to a slight extent , leading to wear and considerable reduction in durability.

A material of a substrate used for a carrier is not particular limited but is preferably any of steel, stainless steel, aluminum, an aluminum alloy, a resin, a fiber-reinforced resin, and a composite thereof. In particular, in polishing a silicon wafer of 300 mm in diameter, it is preferable to use a metal material such as steel, stainless steel, or an aluminum alloy as the material of the substrate.

In the present invention, it is indispensable to completely cover the front and rear surfaces and side surface (section along the thickness direction) of the carrier with a DLC thin film. As mentioned above, a feature of the DLC resides in its structure in which a diamond structure (SP³ bond) and a graphite structure (SP² bond) coexist, and which has a hardness of 1,000 to 4,000 kgf/mm² in terms of Vickers hardness, that is, a high hardness comparable with superhard ceramics or diamond. Meanwhile, the DLC is amorphous, not microcrystalline unlike diamond or superhard ceramics and thus is featured by its ability to form a uniform and highly smooth surface. To elaborate, a feature of the DLC resides in its high hardness equivalent to diamond s well as high surface smoothness and low friction coefficient unlike diamond, in short, its extremely high sliding property. Accordingly, a feature of the carrier for holding a polishing object whose entire surface is coated with the DLC according to the present invention resides in less wear and damage in a sliding portion, and still less wear of the surface. In addition, the entire surface of the carrier of the present invention is coated with the DLC as a nonmetal material, so even with the substrate made of metal, for example, steel, the carrier is free of elution due to a chemical reaction with acid or alkali in the abrasive composition, which enables protection against heavy metal contamination.

As a manufacturing method for the carrier for holding the polishing object, plasma CVD is employed as discussed above. With this method, the entire structure is exposed to plasma, making it possible to cover the entire surface with the uniform and dense DLC coating film. The DLC coating film thickness can be controlled based on coating conditions and time. The carrier is relatively large when taking into account a material size treatable with conventional plasma CVD methods. It is necessary to support the carrier with any holding jig during the treatment, so the supported portion is not exposed to plasma during the treatment. As a result, part of the substrate surface remains untreated. Accordingly, a supporting position is shifted as appropriate, which leaves substantially no uncoated area, and the substantially uniform DLC thin film with the uniform thickness can be formed.

In manufacturing the carrier for holding the polishing object, the substrate thickness is made uniform in advance of the DLC coating on the substrate surface. If the substrate thickness is not uniform, the wear proceeds concentratedly in the thick portion and a coating on this portion peels off. A relative standard deviation of the thickness is preferably 0.4% or less, more preferably 0.2% or less. As a method of uniformizing the substrate thickness, lapping and/ or polishing may be adopted without any particular limitations; alternatively, those may be carried out in turn stepwise.

The DLC coating film thickness ranges from 0.1.µm to 20 µm. With the thickness smaller than 0.1µm, its frictional strength is a little inferior, while the thickness exceeding 20 µm requires much time to form the film and is disadvantageous in terms of cost performance. Also, for the purpose of protecting and surfaces of the polishing object during processing, a resin may be applied to surround the holding hole for holding the polishing object in the DLC coated carrier.

Hereinafter, the method will be described in more detail based on examples and comparative examples, but the present invention is not limited by those examples. A polishing apparatus and conditions used in the examples and comparative examples are summarized in Tables 1 and 3 below. As the polishing apparatus, a double-sided polishing apparatus (DSM-9B, available from SPEEDFAM Co., Ltd.) was used. As the abrasive cloth, SUBA 800 (available from Rodel Co., Ltd.) was used. An abrasive, Rodel 12371 (available from Rodel Co., Ltd.) was prepared by adding 10 parts of pure water per part of concentrate solution.

### Example 1

### (Production of DLC coated carrier)

An SUS-made carrier substrate, which had been lapped into the thickness of 550µm in advance, was coated with DLC into the thickness of 2.5µm. After the substrate was coated with the DLC up to 1.0µm under the film formation temperature set to 150°C, the supporting position was shifted to resume the deposition by 1.5pm.

### (Wear test and durability test)

The resultant DLC coated carrier was polished under the conditions of Table 1 without holding the polishing object. The diluted abrasive was recycled. A wear was determined by calculating a difference between the thicknesses measured before and after the polishing with a micrometer. The durability was examined based on a visual test.

### Comparative Examples 1 to 3

As a carrier, three types of carriers of an SUS-made carrier, a glass epoxy-resin-made (EG-mde) carrier, and an FR-vinylon-made resin were used, none of which were coated with the DLC.

The three types of carriers all had the thickness of about 550µm. The carriers were polished under the conditions of Table 1 without holding a polishing object as in Example 1. The diluted abrasive was recycled.

Polishing test results of Example 1 and Comparative Examples 1 to 3 are listed in Table 2 below.

**Table 1**

| item | processing time |
|---|---|
| processing condition | DSM - 9B |
| platen rotational speed | 100 rpm |
| contact pressure | 70g / cm2 |
| PAD | suba 800 |
| slurry | Rodel 2371 |
| processing time | DLC: 10 hours others: 1 hour |

**Table 2**

| item | material | Example 1 | Comparative Example 1 | Comparative Example 2 | Comparative Example 3 |
|---|---|---|---|---|---|
| | | DLC 1 | SUS | EG^{∗}2 | FR-vinylon |
| | | | | | |
| | unit | | | | |
| wear rate | µm / hr | <0.1 | 4.2 | 4.7 | 0.9 |
| visual test | - | 0 | 0 | 0 | X^{∗}3 |

| | | | | | |
|---|---|---|---|---|---|
| *1 The SUS-made carrier surface is coated with DLC with a thickness of 2.5 µm. *2 glass epoxy resin *3 Cracks occur at the base of gear teeth. | | | | | |

**Table 3**

| item | processing condition |
|---|---|
| Processing condition | DSM - 9B |
| platen rotational speed | 100 rpm |
| contact pressure | 70g / cm² |
| PAD | suba 800 |

### Example 2

The DLC coated carrier produced in Example 1 was polished under the conditions of Table 3 with the carrier holding a 6-inch silicon wafer as a polishing object. In Example 2, the diluted abrasive was not recycled.

### Comparative Examples 4 to 6

The three types of carriers used in Comparative Examples 1 to 3, that is the SUS-made carrier, the glass-epoxy-resin-made (EG-made) carrier ,and the FR-vinylon-made carrier were employed. Similar to Example 2, the carriers were polished under the conditions of Table 3, with the carriers holding the 6-inch silicon ware. In Comparative Example 1 to 3, the diluted abrsive was not recycled. Polishing test results of Example 2 and Comparative Examples 4 to 6 are listed in Table 4below.

**Table 4**

| item | Example | Comparative Example 1 | Comparative Example 2 | Comparative Example 3 |
|---|---|---|---|---|
| | DLC^{∗}1 | SUS | EG^{∗}2 | FR-vinylon |
| Fe | 5 or lower | 45 | 5 or lower | 5 or lower |
| Cu | 5 or lower | 8 | 5 or lower | 5 or lower |
| Cr | 5 or lower | 13 | 5 or lower | 5 or lower |

### Comparison between examples and comparative examples

As a result of comparing Example 1 with Comparative Examples 1 to 3, the wear rate is remarkably low in Example 1, and the carrier made of FR-vinylon (Comparative Example 3) prepared by binding glass fibers with vinylon shows the next lower wear rate. After the test, a visual test was carried out to examine how far cracks occur in each carrier. As a result, the carrier of Example 1 subjected to the visual test over 10 hours showed no change. The carriers of Comparative Examples 1 and 2 subjected to the same over 1 hour showed no change, but cracks occurred at the base of gear teeth of the carrier of Comparative Example 3.

### Evaluation of Example 2 and Comparative Examples 4 to 6

The silicon wafer surface polished in Example 2 and Comparative Example 4 to 6 was rinsed with pure water, after which a mixture of nitric acid and hydrofluoric acid was used to dissolve the surface portion of the silicon wafer. Then, heavy metal in the solution was subjected to quantitative analysis with an ICP mass spectroscope to measure a contamination level of the silicon wafer surface with iron, copper, and chromium. As apparent from the result show in Table 4, the carrier of Example 2 and carriers made of a nonmetal material involved less contamination with heavy metal. In other words, the carrier produced by coating the metal substrate with DLC is comparable with nonmetal carriers in terms of the contamination with heavy metal.

As has been described so far, in Comparative Example 1, the wear rate is high and the contamination with heavy metal is observed. In Comparative Example 2, the durability and the contamination with heavy metal fall within an allowable range, while he wear rate is high. In Comparative Example 3, the durability and the contamination with heavy metal fall within an allowable range, while the cracks occur at the base of gear teeth during the test over 1 hour. This reveals that its durability is far from practical.

The DLC coated carrier of the present invention as explained in Examples 1 and 2 excels in every item: wear resistance, durability, and heavy metal contamination.

As mentioned above, the use of the DLC coated carrier can improve the durability and wear resistance of the carrier as expendables and significantly prolong the service life, so the carrier produces beneficial effects from the economical and qualitative points of view. Furthermore, the metal surfaces is coated throughout, whereby even in polishing, heavy metal neither elutes nor spatters, which is highly advantageous for polishing a polishing object such as a silicon wafer extremely weak against contamination with heavy metal and actually producing the same.

## Claims

1. A method for manufacturing a carrier for holding an object to be polished used for a double-sided polishing apparatus, the method comprising subjecting a substrate to lapping and/or polishing to provide the substrate with a uniform thickness and then coating the entire surface of the substrate with diamond-like carbon, wherein the diamond-like carbon is formed into a film having a thickness of from 0.10 µm to 20 µm, said coating step including providing a surface coating apparatus using a plasma CVD method in which an anode electrode and a cathode electrode are arranged face to face in a vacuum chamber, a carrier substrate is interposed between the anode electrode and the cathode electrode, and RF power is applied between the anode electrode and the cathode electrode while supplying a material gas to form a diamond-like carbon film on the substrate surface; interposing a substrate between the anode electrode and the cathode electrode; and shifting a supporting position of the substrate during formation of the diamond-like carbon film or between current formation and next formation.

2. The method for manufacturing a carrier for holding an object to be polished used for a double-sided polishing apparatus of claim 1, wherein the relative standard deviation of the substrate thickness is no more than 0.4%.

3. The method for manufacturing a carrier for holding an object to be polished used for a double-sided polishing apparatus of claim 1, wherein the substrate is made of a material selected from the group consisting of stainless steel, steel, aluminum, an aluminum alloy, a resin, a fiber-reinforced resin, and a composite thereof.

4. The method for manufacturing a carrier for holding an object to be polished used for a double-sided polishing apparatus according to anyone of claims 1 to 3, wherein the carrier has a holding hole for holding the object and resin portion surrounding the holding hole.

5. A method of manufacturing the object to be polished, the method comprising:
mounting a carrier for holding the object to be polished manufactured according to anyone of claims 1 to 4, between upper and lower platens covered with abrasive cloth;
holding the object to be polished in a holding hole in the carrier for holding the object to be polished; and
rotating at least one of the upper and lower platens and the object to be polished while supplying an abrasive slurry to a surface to be processed to polish both surfaces of the object to be polished,
wherein the abrasive cloth is made of one selected from the group consisting of sued synthetic leather, polyurethane, and a composite prepared by binding non-woven cloth with polyurethane.

## Patentansprüche

1. Verfahren zur Herstellung eines Trägers zum Halten eines zu polierenden Gegenstandes, der für einen doppelseitigen Polierapparat verwendet wird, wobei das Verfahren umfasst, dass ein Substrat Läppen und/oder Polieren ausgesetzt wird, um das Substrat mit einer einheitlichen Dicke zur Verfügung zu stellen, und dann die gesamte Oberfläche des Substrats mit diamantartigem Kohlenstoff beschichtet wird, wobei der diamantartige Kohlenstoff als ein Film mit einer Dicke von 0,10 µm bis 20 µm ausgeformt wird, wobei das Beschichtungsverfahren umfasst: Bereitstellen einer Oberflächenbeschichtungsvorrichtung unter Verwendung eines Plasma-CVD-Verfahrens, bei welchem eine Anodenelektrode und eine Kathodenelektrode in einer Vakuumkammer gegenüber angeordnet sind, ein Trägersubstrat zwischen die Anodenelektrode und die Kathodenelektrode eingeschoben wird, und RF-Energie zwischen die Anodenelektrode und die Kathodenelektrode angelegt wird, während ein Materialgas zugeführt wird, unter Bildung eines diamantartigen Kohlenstofffilms auf der Substratoberfläche; Einschieben eines Substrats zwischen die Anodenelektrode und die Kathodenelektrode; und Verschieben einer Lagerposition des Substrats während der Bildung des diamantartigen Kohlenstofffilms oder zwischen der aktuellen Bildung und der nächsten Bildung.

2. Verfahren zur Herstellung eines Trägers zum Halten eines zu polierenden Gegenstandes, der für einen doppelseitigen Polierapparat verwendet wird, gemäß Anspruch 1, wobei die relative Standardabweichung der Substratdicke nicht mehr als 0,4% ist.

3. Verfahren zur Herstellung eines Trägers zum Halten eines zu polierenden Gegenstandes, der für einen doppelseitigen Polierapparat verwendet wird, gemäß Anspruch 1, wobei das Substrat aus einem Material, ausgewählt aus der Gruppe, bestehend aus Edelstahl, Stahl, Aluminium, einer Aluminiumlegierung, einem Harz, einem faserverstärktem Harz, und einem Verbundwerkstoff davon, hergestellt ist.

4. Verfahren zur Herstellung eines Trägers zum Halten eines zu polierenden Gegenstandes, der für einen doppelseitigen Polierapparat verwendet wird, gemäß einem der Ansprüche 1 bis 3, wobei der Träger eine Halteöffnung zum Halten des Gegenstandes und ein Harzteil, dass die Halteöffnung umgibt, besitzt.

5. Verfahren zur Herstellung des zu polierenden Gegenstandes, wobei das Verfahren umfasst:
Montieren eines Trägers zum Halten des zu polierenden Gegenstandes, der gemäß einem der Ansprüche 1 bis 4 hergestellt wurde, zwischen oberen und unteren Platten, die mit Schleifgewebe beschichtet sind;
Halten des zu polierenden Gegenstandes in einer Halteöffnung in dem Träger zum Halten des zu polierenden Gegenstandes; und
Rotieren zumindest einer der oberen und unteren Platten und des zu polierenden Gegenstandes, während ein Schleifschlamm einer zu behandelnden Oberfläche zugeführt wird, um beide Oberflächen des zu polierenden Gegenstandes zu polieren,
wobei das Schleifgewebe aus einem, ausgewählt aus der Gruppe, bestehend aus synthetischem Wildleder, Polyurethan und einem Verbundwerkstoff, der durch Verbinden von Fließstoff mit Polyurethan hergestellt wird, hergestellt ist.

## Revendications

1. Procédé de fabrication d'un support de maintien d'un objet à polir utilisé pour un appareil de polissage double face, le procédé comprenant l'étape consistant à soumettre un substrat à un rodage et/ou un polissage pour donner au substrat une épaisseur uniforme puis à revêtir la surface entière du substrat avec du carbone de type diamant, dans lequel le carbone de type diamant est formé en un film ayant une épaisseur de 0,10 µm à 20 µm, l'étape de revêtement comprenant la fourniture d'un appareil de revêtement de surface utilisant un procédé de CVD au plasma, dans lequel une électrode d'anode et une électrode de cathode sont disposées face à face dans une chambre sous vide, un substrat porteur est interposé entre l'électrode d'anode et l'électrode de cathode, et une puissance RF est appliquée entre l'électrode d'anode et l'électrode de cathode tout en fournissant un gaz de matériau pour former un film de carbone de type diamant sur la surface du substrat; interposer un substrat entre l'électrode d'anode et l'électrode de cathode et déplacer une position supportante du substrat pendant la formation du film de carbone de type diamant ou entre la formation courante et la formation suivante.

2. Procédé de fabrication d'un support de maintien d'un objet à polir utilisé pour un appareil de polissage double face selon la revendication 1, dans lequel l'écart type relatif de l'épaisseur de substrat n'est pas supérieur à 0,4 %.

3. Procédé de fabrication d'un support de maintien d'un objet à polir utilisé pour un appareil de polissage à double face selon la revendication 1, dans lequel le substrat est constitué d'un matériau choisi dans le groupe consistant en l'acier inoxydable, l'acier, l'aluminium, un alliage d'aluminium, une résine, une résine renforcée de fibres, et un composite de ces derniers.

4. Procédé de fabrication d'un support de maintien d'un objet à polir utilisé pour un appareil de polissage double face selon l'une quelconque des revendications 1 à 4, dans lequel le support contient un trou de maintien permettant de maintenir l'objet et une portion de résine entourant le trou de maintien.

5. Procédé de fabrication de l'objet à polir, le procédé comprenant les étapes consistant à:
monter un support pour maintenir l'objet à polir fabriqué selon l'une quelconque des revendications 1 à 5, entre des plateaux supérieur et intérieur recouverts d'une toile abrasive;
maintenir l'objet à polir dans un trou de maintien dans le support afin de maintenir l'objet à polir; et
mettre en rotation au moins un des plateaux supérieur et inférieur et l'objet à polir tout en alimentant une boue abrasive à une surface à traiter afin de polir les deux surfaces de l'objet à polir,
dans lequel la toile abrasive est constituée d'un élément choisi dans le groupe consistant en le cuir suédé synthétique, le poly(uréthane) et un composite préparé en liant une toile non tissée à du poly(uréthane).
